# EUROPÄISCHE PATENTANMELDUNG

(11) **EP 0 698 598 A2**
(43) Veröffentlichungstag der Anmeldung: **28.02.1996**
(21) Anmeldenummer: 95113113.5
(22) Anmeldetag: 21.08.1995
(51) Int. Cl.: C07C 231/04

(54) **Verfahren zur Herstellung von Amiden**

(30) Priorität: 22.08.1994 DE 4429759
(71) Anmelder: BASF AKTIENGESELLSCHAFT, D-67056 Ludwigshafen (DE)
(72) Erfinder: Eckes, Peter, Dr., D-67166 Otterstadt (DE)
(74) Vertreter: Geissler, Bernhard, Dr.jur., Dipl.-Phys.

(57) **Zusammenfassung**

Die Erfindung betrifft ein Verfahren zur Herstellung von Amiden durch Umsetzung von Fettsäurediketenen mit Aminen, das dadurch ausgezeichnet ist, daß man die Umsetzung in einem Lösungsmittel durchführt, das mindestens ein protisches Lösungsmittel enthält. Bevorzugt werden als Amine Aminopolyole eingesetzt.

## Beschreibung

Die Erfindung betrifft ein Verfahren zur Herstellung von Amiden durch Umsetzung von Fettsäurediketenen mit Aminen.

Im Stand der Technik ist die Umsetzung von Diketen mit Aminverbindungen zu den entsprechenden Amiden bekannt. S. S. Sabri und M. M. El-Abadelah, W. M. Owais, J. Chem. Eng. Data 29, 229 - 231, (1984) beschreiben die Umsetzung von Diketen mit Aminhydrochlorid in methanolischer NaOH und von Diketen mit D-(+)-Glucosamin in wäßriger Natriumhydrogencarbonatlösung. Die Druckschriften CH-PS 619 456, CH-PS 619 457, CH-PS 619 458, J6 3280-048-A, J5 8131-951-A, J6 2149-670-A und EP-A-0 288 628 offenbaren die Umsetzung von Diketen mit Ethanolamin in Methanol oder Ethanol zum entsprechenden Amid. Aus C. Di Bello, F. Filira, V Giormani und F. D'Angeli, J. Chem. Soc. (C), 350 -352 (1969), JA-4716417 und EP-B-0 130 464 ist die Umsetzung von Diketen mit Aminosäuren in wäßrigem Medium oder basischer Lösung, z. B. basischem Methanol, bekannt. Des weiteren erwähnt DT 2166-911 die Umsetzung von Diketen mit einer Diaminobenzol-disulfonsäure in wäßrigem Medium zum entsprechenden Amid.

WO 87/06236 offenbart die Herstellung von oberflächenaktiven Agenzien mittels Umsetzung von Fettsäurediketenen mit Aminopolyolen. Wesentlich ist hierbei, daß die Umsetzung in aprotischen Lösungsmitteln, z. B. Dimethylformamid oder Dimethylsulfoxid, und in Abwesenheit von Wasser stattfinden muß. Hierbei wurde eine Ausbeute von 65% an Endrohprodukt erzielt.

Somit bestand die Aufgabe der vorliegenden Erfindung darin, ein Verfahren zur Herstellung von Amid mit hoher Ausbeute an dem Amid zu schaffen.

Überraschenderweise wurde nun gefunden, daß sich Fettsäurediketene mit Aminen in Anwesenheit von protischen Lösungsmitteln chemoselektiv zu den entsprechenden Amiden umsetzen. Bei Verwendung eines Aminoalkohols als Aminkomponente ergibt sich dabei folgende Reaktionsgleichung: 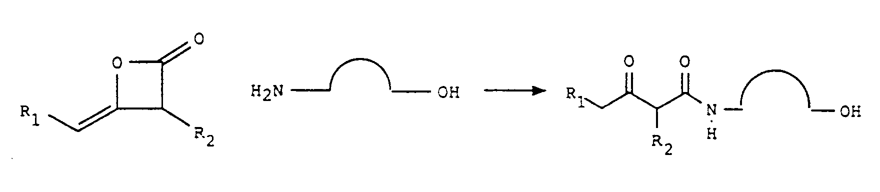
wobei R₁ und R₂, die gleich oder verschieden sein können, Kohlenstoffreste sind, die sich von Carbonsäuren ableiten, die in α-Position mindestens ein Wasserstoffatom besitzen. Vorzugsweise sind R₁ und R₂ gesättigte oder ein- oder mehrfach ungesättigte C₂-C₂₈-Alkylgruppen, die auch verzweigt sein können, insbesondere C₂-C₁₆-Alkylgruppen. Bei dem "Halbkreis" des Aminoalkohols handelt es sich um eine beliebige Kette oder einen beliebigen Cyclus, die/der mit Heteroatomen versehen und substituiert sein kann und ggf. weitere funktionelle Gruppen enthält.

Somit betrifft die Erfindung ein Verfahren zur Herstellung von Amiden durch Umsetzung von Fettsäurediketenen mit Aminen, das dadurch gekennzeichnet ist, daß man die Umsetzung in einem Lösungsmittel durch-führt, das mindestens ein protisches Lösungsmittel enthält. Vorzugsweise enthält das Lösungsmittel 10 bis 100 Gew.-% protisches Lösungsmittel. Weitere bevorzugte Ausführungsformen der Erfindung sind in den Unteransprüchen enthalten.

Das erfindungsgemäß bevorzugte Lösungsmittel enthält 10 bis 100 Gew.-%, insbesondere 50 bis 100 Gew.-%, mindestens eines protischen Lösungsmittels. Definitionsgemäß sind protische Lösungsmittel gemäß Römpp Chemie Lexikon, 9. Auflage, 1991, S. 3660, solche Lösungsmittel, die Protonen enthalten oder freisetzen und/oder Wasserstoff-Brückenbindungen ausbilden können. Besonders bevorzugt ist ein Lösungsmittel, das nur ein oder mehrere protische Lösungsmittel enthält. In einer anderen bevorzugten Ausführungsform enthält das erfindungsgemäß verwendete Lösungsmittel neben dem protischen Lösungsmittel mindestens ein aprotisches Lösungsmittel. Dieses dient zur Lösung des Diketens, da z. B. Diketene mit größeren Kettenlängen fest sind. Aprotische Lösungsmittel sind nach Römpp Chemie Lexikon, 9. Auflage, 1989, S. 232, definiert als nichtwäßrige Lösungsmittel, die kein ionisierbares Proton im Molekül enthalten.

Grundsätzlich eignen sich als protische Lösungsmittel alle protischen Lösungsmittel, wie sie oben definiert wurden. Vorzugsweise verwendet man als protische Lösungsmittel Wasser, C₁-C₈-Alkohole, insbesondere C₁-C₆-Alkohole, Ethylenglykol, Diethylenglykol, Triethylenglykol oder Mischungen davon, wobei insbesondere Wasser und/oder Ethanol oder Wasser und/oder Methanol bevorzugt sind. Von den C₁-C₆-Alkoholen sind Methanol, Ethanol, Isopropanol, n-Butanol oder sek.-Butanol bevorzugt. Des weiteren eignen sich als Lösungsmittel Zweiphasensysteme aus Wasser als protischem Lösungsmittel und mindestens einer Verbindung ausgewählt aus Toluol, Xylol, Hexan, Heptan, Essigester, Methyl-tert.butylether und Methylenchlorid. Diese Zweiphasensysteme enthalten bevorzugt 10 bis 90 Gew.-% Wasser. Auch mit Wasser mischbare Lösungsmittel können verwendet werden, z. B. Tetrahydrofuran (THF) oder Dioxan, das bevorzugt 10 bis 90 Gew.-% protisches Lösungsmittel enthält. Als aprotisches (nicht-protisches) Lösungsmittel sind prinzipiell alle oben definierten aprotischen Lösungsmittel geeignet.

In einer bevorzugten Ausführungsform des erfindungsgemäßen Verfahrens werden Fettsäurediketene gemäß der Formel 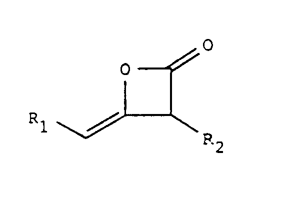
verwendet, wobei R₁ und R₂, die gleich oder verschieden sein können, Kohlenstoffreste sind, die sich von Carbonsäuren ableiten, die in α-Position mindestens ein Wasserstoffatom besitzen. Vorzugsweise sind R₁ und R₂ gesättigte oder ein- oder mehrfach ungesättigte C₂-C₂₈-Alkylgruppen, die auch verzweigt sein können, insbesondere C₂-C₁₆-Alkylgruppen. Besonders bevorzugte Fettsäurediketene sind Hexyldiketen (R₁ und R₂ gleich) oder Octyldiketen (R₁ und R₂ gleich). Verfahren zur Herstellung der erfindungsgemäß geeigneten Fettsäurediketene sind dem Fachmann geläufig; z.B. beschreiben DE-OS 23 35 488 und DE-PS 29 27 118 die Herstellung von Fettalkyldiketenen (Fettsäurediketenen) aus Fettsäurehalogeniden. Die Herstellung von Fettsäurehalogeniden ist z. B. in DE-OS 40 12 781 beschrieben.

Erfindungsgemäß eignen sich als Aminkomponente primäre und sekundäre Amine. Vorzugsweise werden mono- oder polyfunktionelle Amine, insbesondere Aminoalkohole oder Aminopolyole, verwendet. Am meisten bevorzugt sind Aminopolyole, hiervon insbesondere Aminozucker, wie z. B. N-Methylglucamin oder Aminoalkohole, die zwischen der Amin- und Hydroxylgruppe eine beliebige Kette oder einen beliebigen Cyclus aufweisen, die/der mit Heteroatomen versehen und substituiert sein kann und ggf. weitere funktionelle Gruppen enthält. Verfahren zur Herstellung erfindungsgemäß geeigneter Amine sind im Stand der Technik bekannt, siehe z.B. US-A-2,703,798, EP-A-0 220 676, WO 92/08687 und WO 92/06984.

In einer bevorzugten Ausführungsform der Erfindung beträgt das molare Verhältnis von Amin zu Fettsäurediketen 0,1 : 1 bis 10 : 1, insbesondere 0,7 : 1 bis 1,3 : 1. Die Konzentration an Amin beträgt bevorzugt 1 bis 200 Gewichtsteile, insbesondere 5 bis 100 Gewichtsteile, jeweils bezogen auf 100 Gewichtsteile Lösungsmittel. Die Konzentration an Fettsäurediketen liegt vorzugsweise im Bereich von 1 bis 200 Gewichtsteile, insbesondere 5 bis 100 Gewichtsteile, jeweils bezogen auf 100 Gewichtsteile Lösungsmittel. Erfindungsgemäß erfolgt die Umsetzung vorzugsweise bei einer Temperatur von -20 bis 120 °C, insbesondere zwischen 10 und 60 °C. Bei der Umsetzung ist ein Druck von 1 bis 10 bar, besonders 1 bar oder Normaldruck, bevorzugt. Die einzelnen Komponenten werden gleichzeitig oder nacheinander dem Reaktionsgefäß zugesetzt. Die für die spezifischen Komponenten geeigneteste Reihenfolge der Zugabe ist dem Fachmann geläufig.

Die nach dem erfindungsgemäßen Verfahren hergestellten Amide finden insbesondere Verwendung als oberflächenaktive Stoffe (Tenside). Das erfindungsgemäße Verfahren bietet den Vorteil, daß in organischen Lösungsmitteln nicht oder nur schlecht lösliche Amine verwendet werden können.

Die nachfolgenden Beispiele erläutern die Erfindung.

### Beispiel 1

Gemäß folgender Tabelle wird in einem Dreihalskolben mit Rührer, Thermometer und Rückflußkühler zu einer Lösung enthaltend Diethylamin, Ethanol und Methyl-t-butylether (MTBE), in einer Stunde bei Raumtemperatur Hexyldiketen gegeben. Man läßt die Reaktionsmischung 1 Stunde bei Raumtemperatur nachrühren und untersucht das Rohprodukt mittels Gaschromatographie.

| Diethylamin g (mmol) | Ethanol g (mmol) | Hexyldiketen g (mmol) | MTBE ml | Gehalt % (Flächen % im GC) Amid |
|---|---|---|---|---|
| 1,10 (15) | 0,69 (15) | 2,06 (10) | 10 | 96 |
| 1,10 (15) | 6,9 (150) | 2,06 (10) | 10 | 96 |
| 2,19 (30) | 138 (3000) | 3,96 (20) | - | 98 |

### Beispiel 2

Gemäß folgender Tabelle wird in einem Dreihalskolben mit Rührer, Thermometer und Rückflußkühler zu einer Lösung enthaltend Diethylamin, Wasser und THF in einer Stunde bei Raumtemperatur Hexyldiketen zugegeben. Man läßt die Reaktionsmischung 1 Stunde bei Raumtemperatur nachrühren und untersucht das Rohprodukt mittels Gaschromatographie.

| Diethylamin g (mmol) | Wasser g (mmol) | Hexyldiketen g (mmol) | MTBE ml | Gehalt % (Flächen% im GC) Amid |
|---|---|---|---|---|
| 2,19 (30) | 0,54 (30) | 3,96 (20) | 20 | 98 |
| 2,19 (30) | 5,4 (300) | 3,96 (20) | 20 | 98 |
| 2,19 (30) | 54 (3000) | 3,96 (20) | - | 98 |

### Beispiel 3

29,3 g N-Methylglucamin werden in 400 ml Wasser bei 50 °C vorgelegt. Zu der klaren wäßrigen Lösung gibt man in 1 Stunde bei 50 °C 33,6 g Hexyldiketen (Gehalt 92%) und läßt noch 1 Stunde bei gleicher Temperatur nachrühren. Nach Entfernen des Wassers im Vakuum wird das Rohprodukt aus 1500 ml Essigester umkristallisiert. Es werden 50,5 g (86%) 2-Butyl-3-oxo-octansäure-methyl-((2S,3S,4S,5R)-2,3,4,5,6-penta-hydroxy-hexyl)-amid als weißer Feststoff mit einem Schmelzpunkt von 118 - 120 °C und einer Hydroxylzahl von 708 mg KOH/g erhalten.

### Beispiel 4

33,0 g N-Methylglucamin werden in 400 ml Wasser gelöst. Zu der Lösung tropft man in 3,5 Stunden eine Lösung von 40 g Hexyldiketen (Gehalt 92%) in 100 ml Hexan. Die resultierende Suspension wird 1 Stunde bei 50 °C gerührt und der Feststoff nach Kühlen auf Raumtemperatur durch Filtration abgetrennt. Das Rohprodukt wird durch Kristallisation in Essigester gereinigt. Man erhält 54,2 g (81%) des gleichen Amids wie in Beispiel 3 als weißen Feststoff mit den gleichen analytischen Daten.

### Beispiel 5

33,0 g N-Methylglucamin werden in 400 ml Ethanol suspendiert. Bei Raumtemperatur läßt man zu der Suspension 39,7 g Hexyldiketen (Gehalt 92%) in 1 Stunde zutropfen. Nach Erwärmen auf 50 °C rührt man eine Stunde. Die klare homogene Lösung wird im Vakuum vom Lösungsmittel befreit und das Rohprodukt aus Essigester umkristallisiert. Es werden 53,6 g (81%) des gleichen Amids wie in Beispiel 3 als weißer Feststoff erhalten. Die analytischen Daten entsprechen denen aus Beispiel 3.

### Beispiel 6

29,3 g N-Methylglucamin werden in 400 ml Wasser gelöst und zu der Lösung in 2,5 Stunden eine Lösung von 42,5 g Octyldiketen (Gehalt 93%) in 150 ml Hexan getropft. Die Emulsion wird 1 Stunde bei Raumtemperatur gerührt und von Lösungsmittel befreit. Chromatographische Reinigung ergibt 58,6 g (87%) 2-Hexyl-3-oxo-decansäure-methyl-((2S,3S,4S,5R)-2,3,4,5,6-pentahydroxy-hexyl)-amid als analysenreinem Feststoff.

### Beispiel 7

29,3 g N-Methylglucamin suspendiert man in 400 ml Ethanol bei Raumtemperatur und gibt in 3 Stunden 42,5 Octyldiketen (Gehalt 93%) zu. Die homogene Lösung wird noch 1 Stunde bei 50 °C gerührt. Das Ethanol entfernt man im Vakuum und reinigt das Rohprodukt durch Chromatographie an Kieselgel. Man erhält 53,3 g (79%) des gleichen Amids wie in Beispiel 6, wobei die analytischen Daten denen aus Beispiel 6 entsprechen.

Die Beispiele zeigen deutlich, daß das erfindungsgemäße Verfahren zu einer höheren Ausbeute an Amid führt als im Stand der Technik bekannte Verfahren mit 65% Ausbeute.

## Patentansprüche

1. Verfahren zur Herstellung von Amiden durch Umsetzung von Fettsäurediketenen mit Aminen, **dadurch gekennzeichnet**, daß man die Umsetzung in einem Lösungsmittel durchführt, das mindestens ein protisches Lösungsmittel enthält.

2. Verfahren nach Anspruch 1, dadurch gekennzeichnet, daß das Lösungsmittel 10 bis 100 Gew.-%, insbesondere 50 bis 100 Gew.-%, mindestens eines protischen Lösungsmittels enthält.

3. Verfahren nach Anspruch 1 oder 2, dadurch gekennzeichnet, daß man als protische Lösungsmittel Wasser, C₁-C₈-Alkohole oder Mischungen davon verwendet.

4. Verfahren nach Anspruch 1 oder 2, dadurch gekennzeichnet, daß man als Lösungsmittel Zweiphasensysteme aus Wasser als protischem Lösungsmittel und mindestens einer Verbindung ausgewählt aus Toluol, Xylol, Hexan, Heptan, Methyl-tert.-butylether, Essigester oder Methylenchlorid verwendet.

5. Verfahren nach einem der vorhergehenden Ansprüche, dadurch gekennzeichnet, daß man Fettsäurediketene der Formel 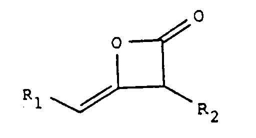 verwendet, wobei R₁ und R₂, die gleich oder verschieden sein können, gesättigte oder ein- oder mehrfach ungesättigte C₂-C₂₈-Alkylgruppen, die auch verzweigt sein können, insbesondere C₂-C₁₆-Alkylgruppen, sind.

6. Verfahren nach einem der vorhergehenden Ansprüche, dadurch gekennzeichnet, daß man Hexyldiketen oder Octyldiketen einsetzt.

7. Verfahren nach einem der vorhergehenden Ansprüche, dadurch gekennzeichnet, daß man als Amine mono- oder polyfunktionelle Amine, insbesondere Aminopolyole, verwendet.

8. Verfahren nach einem der Ansprüche 1 bis 6, dadurch gekennzeichnet, daß man als Amin Aminozucker, insbesondere N-Methylglucamin verwendet.

9. Verfahren nach einem der vorhergehenden Ansprüche, dadurch gekennzeichnet, daß das molare Verhältnis von Amin zu Fettsäurediketen 0,1 : 1 bis 10 : 1, insbesondere 0,7 : 1 bis 1,3 : 1, beträgt.

10. Verfahren nach einem der vorhergehenden Ansprüche, dadurch gekennzeichnet, daß die Konzentration an Amin 1 bis 200 Gewichtsteile und die Konzentration an Fettsäurediketen 1 bis 200 Gewichtsteile beträgt, jeweils bezogen auf 100 Gewichtsteile Lösungsmittel.
